# EUROPÄISCHE PATENTSCHRIFT

(11) **EP 0 531 614 B1**
(45) Veröffentlichungstag und Bekanntmachung des Hinweises auf die Patenterteilung: **26.06.1996**
(21) Anmeldenummer: 92107001.7
(22) Anmeldetag: 24.04.1992
(51) Int. Cl.: G01N 33/00, G01N 27/16, G01N 27/04

(54) **Gasmessgerät**
Gas measuring apparatus
Dispositif de detection de gaz

(30) Priorität: 11.09.1991 DE 4130099
(43) Veröffentlichungstag der Anmeldung: 17.03.1993
(73) Patentinhaber: AUERGESELLSCHAFT GMBH, D-12059 Berlin (DE)
(72) Erfinder: Weidinger, Joachim, W-1000 Berlin 10 (DE); Axel, Schubert, Dr., W-1000 Berlin 49 (DE)

(56) Entgegenhaltungen:
- EP-A- 0 440 906
- WO-A-82/03689
- DE-A- 3 127 431
- DE-A- 3 615 876

## Beschreibung

Die Erfindung betrifft ein Gasmeßgerät nach dem Oberbegriff des Anspruchs 1.

Ein Gasmeßgerät beruhend auf der Messung mit einer Meßbrücke nach dem Wärmetönungs- oder Wärmeleitfähigkeits prinzip ist bekannt aus der EP-A- 0 440 906.

Bei einem derartigen Gasmeßgerät wird die zu untersuchende Meßgasprobe von einer elektrischen Meßgasförderpumpe durch eine Verbrennungskammer gesaugt, wo ein Teil der brennbaren Anteile der Probe an einem vorgeheizten Meßsensor verbrannt wird. Die katalytische Verbrennung (Wärmetönung) ergibt ein Ausgangssignal am Sensor, dessen Maximum der Konzentration des Gases entspricht. Um dem Gerätträger die Gewißheit zu geben, daß die Gasprobe die Verbrennungskammer erreicht und diese durchspült, oder nicht, ist es erforderlich, daß bei der Meßgasförderung der Volumenstrom überwacht und angezeigt werden kann.

Es ist bekannt, eine derartige überwachung mittels Drucksensoren zu verwirklichen, die in der Brennkammer im Strömungsweg des Meßgases angeordnet sind und eine Druckerhöhung als Maß für den Volumenstrom anzeigen, während bei Nichtvorhandensein eines Druckes dies ein Indiz dafür ist, daß die Meßgasförderung unterbrochen ist. Die Verwendung von zusätzlichen Drucksensoren mit einer erforderlichen Schaltungsanordnung zur Auswertung, ist schaltungstechnisch und von den Kosten her sehr aufwendig.

Der Erfindung liegt die Aufgabe zugrunde, ein Gasmeßgerät mit einer Überwachung des Volumenstroms einer Meßgasförderpumpe der eingangs genannten Art zu entwickeln, die mit einfachen schaltungstechnischen Mitteln eine sichere Überwachung gewährleistet.

Die mit der Erfindung erzielten Vorteile bestehen insbesonders darin, daß zur Überwachung die ohnehin für die Messung von Gaskonzentrationen vorhandenen Wärmetönungs- oder Wärmeleitfähigkeitssensoren mit benutzt werden und somit keine zusätzlichen Meßelemente für den Überwachungsmeßkreis erforderlich sind.

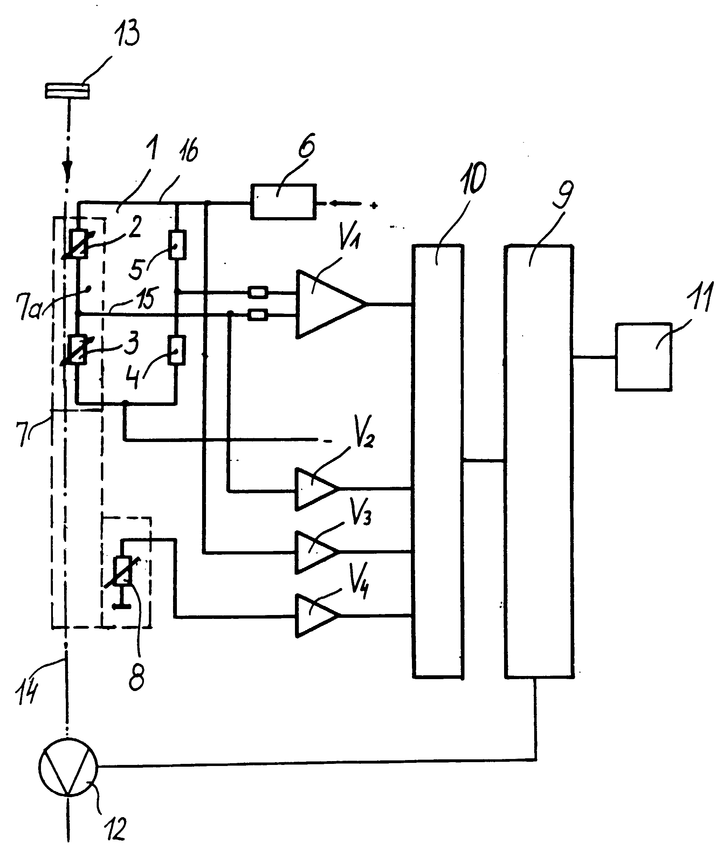

Ein Ausführungsbeispiel der Erfindung ist in der Zeichnung dargestellt und wird im folgenden näher beschrieben.

Die einzige Figur zeigt eine erfindungsgemäße Schaltungsanordnung zur Überwachung des Volumenstroms der Meßgasförderung. In einer Meßbrücke 1 sind ein katalytisch aktiver Meßsensor 2 als Meßelement und ein katalytisch inaktiver Sensor 3 als Kompensationselement sowie zwei Festwiderstände 4 und 5 angeordnet. Der katalytisch aktive Meßsensor 2 entspricht in seiner Wirkung einem Wärmetönungssensor und der katalytisch inaktive Sensor 3 einem Wärmeleitfähigkeitssensor. Die Meßbrücke 1 wird über eine Strom-/Spannungsquelle 6 gespeist, die zum Aufheizen für den Meßsensor 2 und den inaktiven Sensor 3 dient, die in einem Meßkopf 7 in voneinander getrennten, jedoch miteinander in Verbindung stehenden Brennkammerräumen 7a angeordnet sind. Im Meßkopf 7 ist in einer von den Sensoren 2 und 3 abgetrennten Kammer ein Temperatursensor 8 als Kompensationselement für den Einfluß wechselnder Umgebungstemperatur des Gasmeßgerätes oder unterschiedlicher Temperaturen der Meßkomponenten angeordnet. Auf diese Weise ist ein temperaturunabhängiger Betrieb der Volumenstromüberwachung möglich. Dies ist besonders deshalb wichtig, um eine eindeutige Anzeige für die Auswertung des Wärmeleitfähigkeitssignals des katalytisch inaktiven Sensors 3 zu erhalten, der mit zur Volumenstromüberwachung herangezogen wird und in seinem Meßverhalten stark temperaturabhängig ist, was korrigiert werden muß.

Das von der Meßkomponente am Meßsensor 2 erzeugte konzentrationsabhängige Wärmetönungssignal wird im Diagonalzweig 15 der Meßbrücke 1 einem Verstärker V₁ zugeführt, um von der Meßbrücke entkoppelt und verstärkt zur Auswertung an einen Mikrocomputer 9 zu gelangen. Dem Mikrocomputer 9 ist ein AD-Wandler 10 vorgeschaltet, da für die Verarbeitung der analogen Meßsignale durch den Mikrocomputer jedes Signal zuerst in dem AD-Wandler digitalisiert werden muß.

Neben der Erfassung des Wärmetönungs-Meßsignals werden über einen Verstärker V₂ die Signalkomponente am Kompensationssensor 3 und über einen Verstärker V₃ die Signalkomponente am Meßsensor 2 sowie über einen Verstärker V₄ die Signalkomponente am Temperatursensor 8 getrennt erfaßt. Die Ausgangssignale der Verstärker V₂ bis V₄ werden ebenfalls dem AD-Wandler 10 zugeführt, und werden dann mit dem nachgeschalteten Mikrocomputer 9 einzeln durch geeignete Verknüpfung der Signale weiterverarbeitet, um als Meßwerte auf einer dem Mikrocomputer nachgeschalteten LCD-Anzeige 11 zur Anzeige gebracht zu werden. Am Mikrocomputer 9 ist eine Meßgasförderpumpe 12 angeschlossen, die an einer Meßstelle 13 eine Luftprobe entnimmt, die entlang eines Gasweges 14 durch die Brennkammerräume 7a gesaugt wird.

Nachfolgend wird der Ablauf eines ersten Ausführungsbeispiels der erfindungsgemäßen Volumenstromüberwachung beschrieben:

Die Meßbrücke mit dem Meßsensor 2 und dem Kompensationssensor 3 wird eingeschaltet und mit der Strom-/Spannungsquelle 6 auf eine Arbeitstemperatur aufgeheizt, die beispielsweise 400 °C beträgt.

Durch den Mikrocomputer 9 wird - um einen zeitlichen Faktor verzögert - die Meßgasförderpumpe 12 eingeschaltet, so daß über den aufgeheizten Meßsensor 2 und Kompensationssensor 3 ein Volumenstrom geleitet wird. Aufgrund des auftretenden Volumenstroms kühlen sich die aufgeheizten Sensoren 2 und 3 ab, wodurch deren Widerstände sich ändern, und zwar erfolgt eine Widerstandsverringerung der Sensoren. Der charakteristische Kurvenverlauf der Widerstandsänderung an den sich abkühlenden Meß- und Kompensationssensoren 2 und 3 wird über die Verstärker V₂ und V₃ elektrisch weiterverarbeitet und im Mikrocomputer 9 ausgewertet, um dann als ein Meßwert an der LCD-Anzeige 11 angezeigt werden zu können, und zwar, ob ein Volumenstrom durch die Brennkammerräume 7a fließt oder nicht.

Falls keiner fließt und eine Störung im Gasweg vorliegt, kann an den Sensoren keine Abkühlung erfolgen. Diese Erkennung erfolgt aufgrund des Kurvenverlaufs einer im Mikrocomputer 9 gespeicherten Temperaturkennlinie des Meßsensors 2.

Bei einem weiteren Ausführungsbeispiel kann die Auswertung zur Volumenstromüberwachung vorteilhaft derart erfolgen, daß nur der Kompensationssensor 3 aufgeheizt wird, und zwar auf eine Arbeitstemperatur von z.B. 100 °C. Hierdurch wird der Energieverbrauch für die Volumenstrommessung herabgesetzt. Nach der Aufheizung des katalytisch inaktiven Sensors 3 wird die Pumpe 12 in Betrieb genommen. Aufgrund des auftretenden Volumenstroms erfolgt eine Abkühlung des Sensors 3, die über den Verstärker V₂ gemessen und in der bereits beschriebenen Art, wie beim ersten Ausführungsbeispiel, entsprechend weiterverarbeitet und zur Anzeige gebracht werden kann. Ist der Durchfluß des Gasweges gestört, bleibt der Sensor 3 in einem aufgeheizten Zustand. Dies wird als Indiz einer Störung ebenfalls angezeigt.

Die eigentliche Gasmessung erfolgt erst zu einem späteren Zeitpunkt, wenn das Meßgas 14 in die Brennkammerräume 7a gefördert wurde, die Meßgasförderpumpe 12 abgestellt ist und die Verbrennung in der Brennkammer stattfinden kann. Die Signalauswertung erfolgt dann über den Verstärker V₁.

## Patentansprüche

1. Gasmeßgerät zur Messung von brennbaren Gasen und Dämpfen in Luft, beruhend auf der Messung mit einer Meßbrücke nach dem Wärmetönungs- oder Wärmeleitfähigkeitsprinzip und mit einer Anordnung zur Überwachung des mittels einer Meßgasförderpumpe geförderten Volumenstroms durch die Brennkammer eines Meßkopfes, dadurch gekennzeichnet, daß zur Überwachung des Volumenstroms (14) ein in den Meßkreis der Meßbrücke (1) integrierter Überwachungsmeßkreis (V₂, V₃, V₄ und 8) vorgesehen ist, der aus jeweils die Spannungssignale des Wärmetönungssensors (2), des Wärmeleitfähigkeitssensors (3) und eines Temperatursensors (8) getrennt messende Verstärker (V₂, V₃ und V₄) besteht, die an eine Signalverarbeitungsanlage (9, 10 und 11) angeschaltet sind.

2. Verfahren zur Anwendung eines Gasmeßgeräts nach Anspruch 1, dadurch gekennzeichnet, daß der zu überwachende Volumenstrom (14) über die vorgeheizten Wärmetönungs- und Wärmeleitfähigkeitssensoren (2, 3) geleitet wird, und an diesen eine Abkühlung bewirkt, wodurch die Sensoren (2, 3) eine Widerstandsverringerung erfahren, die als Maß für den vorliegenden Volumenstrom gemessen und mit einem in der Signalverarbeitungsanlage (9, 10 und 11) gespeicherten vorgegebenen charakteristischen Kurvenverlauf einer Temperaturkennlinie des Wärmetönungssensors (2) verglichen und als Meßwert an einer Anzeige (11) angezeigt wird.

3. Verfahren zur Anwendung eines Gasmeßgeräts nach Anspruch 1, dadurch gekennzeichnet, daß zur Überwachung des Volumenstroms der Wärmeleitfähigkeitssensor (3) aufgeheizt wird, wobei aufgrund des auftretenden Volumenstroms (14) am Sensor eine Abkühlung erfolgt, die über den am Diagonalzweig (15) der Meßbrücke (1) angeschalteten Verstärker (V₂) gemessen und in der Signalverarbeitungsanlage (9, 10 und 11) weiterverarbeitet wird.

4. Verfahren nach Anspruch 2, dadurch gekennzeichnet, daß die Signalkomponente am Wärmeleitfähigkeitssensor (3) über den am Diagonalzweig (15) geschalteten Verstärker (V₂) und die Signalkomponente am Wärmetönungssensor (2) über den am Brückenzweig (16) geschalteten Verstärker (V₃) sowie die Signalkomponente am Temperatursensor 8 über den Verstärker (V₄) jeweils getrennt erfaßt und einem AD-Wandler (10) zugeführt wird.

## Claims

1. A gas measuring device for the measurement of flammable gases and vapors in the air, based on a measuring bridge following the principle of catalytic combustion or thermal conductivity and with an arrangement for the monitoring of the volume flow induced by a measuring gas feed pump through the combustion chamber of a measuring head, characterised in that, for the monitoring of the volume flow (14) a monitoring measuring circuit (V2 V3, V4 and 8) is integrated into the measuring circuit of the measuring bridge (1), the said measuring circuit comprising amplifiers (V2, V3 and V4) for the separate processing of the voltage signals of the catalytic combustion sensor (2), the thermal conductivity sensor (3) and a temperature sensor (8), whereby the said amplifiers are connected to a signal processing unit (9, 10 and 11).

2. A process for the application of a gas measuring device according to claim 1, characterised in that the volume flow to be monitored (14) is fed across the pre-heated catalytic combustion and thermal conductivity sensors (2, 3), thus causing a cooling effect on them, which lowers the resistance of the sensors (2, 3) which is recorded as a measurement of the volume flow in question, and is compared with one of the curves stored in the signal processing unit (9, 10 and 11), whereas this curve is representative of a temperature curve of the catalytic combustion sensor (2), the measured value obtained is shown on a display (11).

3. A process for the application of a gas measuring device according to claim 1, characterised in that the thermal conductivity sensor (3) is pre-heated for the monitoring of the volume flow, whereas the volume flow (14) at the sensor causes a cooling effect which is measured by the amplifier (V2) connected to the diagonal branch (15) of the measuring bridge (1) and then further processed in the signal processing unit (9, 10 and 11).

4. A process according to claim 2, characterised in that the signal component available at the thermal conductivity sensor (3) is acquired separately via the amplifier (V2) connected to the diagonal branch (15), the signal component available at the catalytic combustion sensor (2) via the amplifier (V3) connected to the bridge branch (16) and the signal component available at the temperature sensor (8) via the amplifier (V4), before being fed into an analogue/digital converter (10).

## Revendications

1. Appareil de mesure de gaz, pour mesurer des gaz et des vapeurs inflammables dans l'air, fondé sur un pont de mesure selon le principe de combustion catalytique ou de conductivité thermique et disposant d'un système de surveillance du débit volumétrique généré par une pompe d'alimentation en gaz de mesure, débit traversant la chambre de combustion d'une tête de mesure, caractérisé en ce que le système de surveillance du débit volumétrique (14) comporte un circuit de surveillance (V2, V3, V4 et 8) intégré au pont de mesure, lequel circuit se compose d'amplificateurs (V2, V3 et V4), mesurant séparément les signaux de tension du détecteur de combustion catalytique (2), du détecteur de conductivité thermique (3) et d'un capteur de température (8), amplificateurs reliés à un dispositif de traitement des signaux (9, 10 et 11).

2. Procédé d'application d'un appareil de mesure de gaz selon la revendication 1, caractérisé en ce que le débit volumétrique à surveiller (14) est transmis à l'aide des capteurs à combustion catalytique et à préchauffés (2, 3), en ce que ledit débit provoque un refroidissement au niveau de ces détecteurs (2,3) qui subissent alors une baisse de résistance qui sert de mesure du présent débit volumétrique, mesure comparée à une courbe caractéristique pour le détecteur à combustion catalytique (2), en mémoire dans le dispositif de traitement des signaux (9, 10 et 11) avant d'être présentée comme valeur de mesure à l'aide d'un système d'affichage (11).

3. Procédé d'application d'un appareil de mesure de gaz selon la revendication 1, caractérisé en ce que le capteur de conductivité thermique (3), destiné à la surveillance du débit volumétrique, est préchauffé, provoquant ainsi le refroidissement du détecteur, à cause de l'apparition du débit volumétrique, lequel refroidissement est mesuré par l'amplificateur (V2) branché à la branche diagonale (15) du pont de mesure (1), avant de subir un nouveau traitement dans le dispositif de traitement des signaux (9, 10 et 11).

4. Procédé d'application d'un appareil de mesure de gaz selon la revendication 2, caractérisé en ce que la composante du signal au niveau du capteur de conductivité thermique (3) est acquise séparément à l'aide de l'amplificateur (V2) connecté à la branche diagonale (15) et que la composante de signal au niveau du capteur de coloration thermique (2) est acquise à l'aide de l'amplificateur (V3) connecté à la branche du pont (16) tandis que la composante de signal au niveau du capteur de température 8 est acquise par l'amplificateur (V4), avant que tous ces signaux ne soient transmis à un convertisseur analogique/numérique (10).
